Europäisches Patentamt

European Patent Office

Office européen des brevets

(19)

(11) Veröffentlichungsnummer: **0 174 525**
**B1**

(12) **EUROPÄISCHE PATENTSCHRIFT**

(45) Veröffentlichungstag der Patentschrift: **08.08.90**

(21) Anmeldenummer: **85110454.7**

(22) Anmeldetag: **20.08.85**

(51) Int. Cl.⁵: **C 07 H 21/00**

(54) **Verfahren zur Aufreinigung synthetischer Oligonucleotide.**

(30) Priorität: **13.09.84 DE 3433649**

(43) Veröffentlichungstag der Anmeldung:
**19.03.86 Patentblatt 86/12**

(45) Bekanntmachung des Hinweises auf die
Patenterteilung:
**08.08.90 Patentblatt 90/32**

(84) Benannte Vertragsstaaten:
**AT BE CH DE FR GB IT LI NL SE**

(56) Entgegenhaltungen:
**EP-A-0 108 387**

**CHEMICAL ABSTRACTS, Band 81, 1974, Seite
201, Zusammenfassung Nr. 60241m, Columbus,
Ohio, US; R.A. HOLTON et al.: "Rapid separation
and isolation of mono- and oligonucleotides by
high-speed liquid chromatography. Ionexchange, reversed-phase system", &
BIOCHEM. BIOPHYS. RES. COMMUN. 1974,
58(3), 605-12**

(73) Patentinhaber: **Gesellschaft für
Biotechnologische Forschung mbH (GBF)
Mascheroder Weg 1
D-3300 Braunschweig-Stöckheim (DE)**

(72) Erfinder: **Blöcker, Helmut, Dr.
Schinkelstrasse 6
D-2000 Hamburg 60 (DE)**
Erfinder: **Frank, Ronald, Dr.
Leibnizstrasse 8
D-3340 Wolfenbüttel (DE)**
Erfinder: **Heisterberg-Moutsis, Gudrun
Steinbachweg 15
D-6900 Heidelberg (DE)**
Erfinder: **Kurth, Gisela
Leibnizstrasse 8
D-3340 Wolfenbüttel (DE)**
Erfinder: **Meyerhans, Andreas, Dipl.-Chem.
Haidkamp 27
D-2080 Pinneberg (DE)**

(74) Vertreter: **Boeters, Hans Dietrich, Dr. et al
Boeters, Bauer & Partner Thomas-Wimmer-Ring
14
D-8000 München 22 (DE)**

Courier Press, Leamington Spa, England.

(56) Entgegenhaltungen:

H.G. GASSEN et al.: "Chemical and enzymatic synthesis of gene fragments. A laboratory manual", 1982, Seiten 9,177-198, Verlag Chemie, Weinheim, DE;

JOURNAL OF THE AMERICAN CHEMICAL SOCIETY, Band 105, Nr. 3, 1983, Seiten 661-663, American Chemical Society, US; S.P. ADAMS et al.: "Hindered dialkylamino nucleoside phosphite reagents in the synthesis of two DNA 51-mers"

Biochim. Biophys. Res. Comm. 58(3),1974, 605-12.

# EP 0 174 525 B1

## Beschreibung

Die Erfindung betrifft ein Verfahren zu Aufreinigung von synthetischen Oligonucleotiden, durch das die bisher sehr langwierige Aufreinigung von synthetischen Oligonucleotiden entscheidend beschleunigt wird.

Die heute bekannten Festphasenmethoden erlauben es, eine große Anzahl verschiedener Oligonucleotide in relativ kurzer Zeit herzustellen.

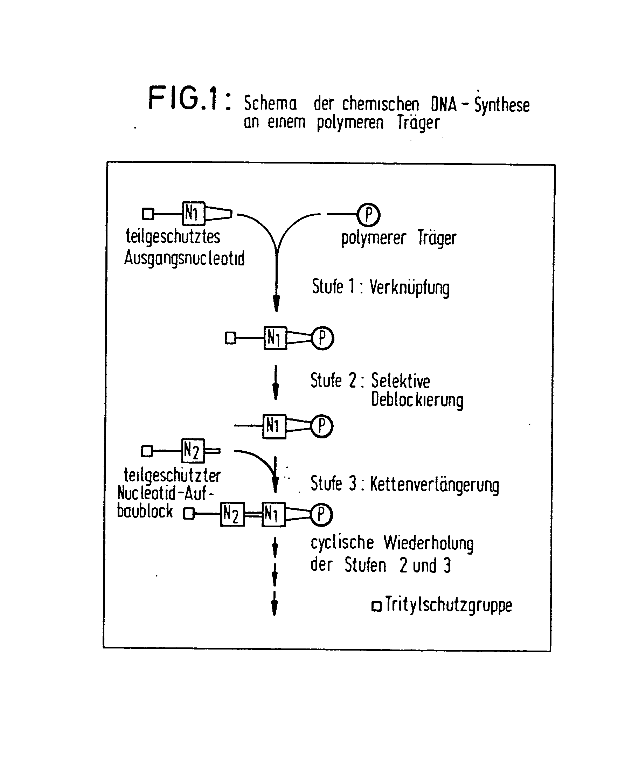

Bei den gängigen Verfahren der chemischen Oligonucleotidsynthese (z.B. auf einem polymeren Träger, siehe Fig. 1) werden 5'-tritylierte Nucleotidbausteine in der gewünschten Reihenfolge schrittweise miteinander verknüpft. Am Ende einer solchen Synthese erhält man neben dem gewünschten, vollständig geschützten Oligonucleotid verschiedene kürzere Fragmente (Abbruchsequenzen), die entweder freie Hydroxylgruppen oder Acetylgruppen (eingeführt durch eine Blockierungsreaktion zwischen den Kettenverlängerungsschritten) und in sehr geringem Maße auch noch Tritylgruppen am 5'-Ende haben.

Je nach verwendeter Synthesemethode (Phosphotriester, Phosphittriester) werden auch andere, modifizierte Oligonucleotide und Fragmente als Nebenprodukte erhalten. Diese Produkte müssen aber einer Reihe von Entschützungs- und Säuberungsschritten unterworfen werden. Dabei übertrifft der Zeitbedarf dieser Aufreinigung bei weitem die Synthesezeit.

Das folgende Schema gibt die nacheinander auszuführenden Reinigungsschritte wieder, wie sie in den meisten DNA-Syntheselabors heute durchgeführt werden (vgl. H. G. Gasser und Anne Lang, "Chemical and Enzymatic Synthesis of Gene Fragments, a Laboratory Manual", Verlag Chemie, Weinheim, 1982):

1. teilweise Entschützung und Abspaltung vom Träger
2. Einengen der Lösung
3. chromatographische Reinigung des tritylierten Oligonucleotids
4. Einengen der Lösung
5. Säure-katalysierte Detritylierung
6. Einengen der Lösung
7. Hochdruck-Flüssigchromatographie (HPLC) oder Polyacrylamid-Gelelektrophorese (PAGE)
8. Entsalzung oder Elution und Entsalzung
9. Einengen der Lösung

In diesen Reinigungsschritten sind die Schritte 2. und 4., nämlich das Einengen der jeweiligen Lösungen, besonders zeitraubend.

In dem Entschützungsschritt 1. werden bis auf die lipophile Tritylgruppe alle anderen Schutzgruppen abgespalten.

Diese Tritylgruppe befindet sich jetzt nur noch am 5'-Ende des gewünschten Oligonucleotids.

Es wurde non überaschenderweise festgestellt, daß die hydrophoben Eigenschaften dieser Tritylgruppe über Wechselwirkung mit "reversed phase"-Material eine Abtrennung von den oben erwähnten Nebenprodukten und abgespaltenen Schutzgruppen ermöglicht.

Die Erfindung betrifft somit ein Verfahren zur Aufreinigung von synthetischen Oligonucleotiden, das dadurch gekennzeichnet ist, daß man

—die gegebenenfalls von einem Träger abgespaltenen, noch mit lipophiler Schutzgruppe versehenen Oligonucleotide ohne Einengen der Abspaltungslösung

—an einen Träger mit Umkehrphaseneigenschaften und gebenenfalls Ionenaustauschereigenschaften adsorbiert,

—die Oligonucleotide ohne lipophile Schutzgruppe entfernt,

—die Schutzgruppe von den adsorbierten Oligonucleotiden abspaltet,

—die adsorbierten Oligonucleotide erneut wäscht,

—die adsorbierten Oligonucleotide vom Träger eluiert und

—danach die erluierten Oligonucleotide in an sich bekannter Weise weiter aufreinigt.

Durch die erfindungsgemäße Kombination eines hydrophoben Adsorptionsmaterial mit reversed phase-Eigenschaften und eines Ionenaustauschers ist erstmals die kombinierte Durchführung der folgenden Schritte in Form einer chromatographiertigen Operation ermöglicht:

—direkte Adsorption des mit Hilfe einer lipophilen Schutzgruppe geschützten Oligonucleotids aus einer Entschützungslösung

—Reinigung des lipophil-geschützten Oligonucleotids

—Abspaltung der lipophilen Schutzgruppe von der adsorbierten Verbindung.

Im erfindungsgemäßen Verfahren werden die Schritte 2. bis 5. des oben angegebenen Schemas in einer chromatographischen Operation zusammengefaßt.

In einer bevorzugten Ausführungsform des erfindungsgemäßen Verfahrens wird die ammoniakalische Entschützungslösung aus Schritt 1. nach Abdampfen des Ammoniaks auf eine Säule aufgegeben, die mit Polytetrafluorethylen (PTFE, vgl. Römpps Chemie-Lexikon, 7. Auflage, S.3478 (1977) und DEAE-Cellulose (vgl. Römpps Chemie-Lexikon, 8. Auflage, S.629 und 874 (1981) gepackt ist, wobei das Polytetrafluorethylen und die DEAE-Cellulose miteinander innig vermischt werden.

Das in der Lösung vorhandene, am 5'-Ende tritylierte Oligonucleotid wird an der festen Phase adsorbiert. Durch einen ersten Waschschritt, vorzugsweise mit einer Triethylammoniumbicarbonat-Lösung, wird ein großer Teil von Schutzgruppen wie auch vorhandene Oligonucleotide ohne Tritylgruppe entfernt (Schritt 3. des oben angegebenen Schemas). Durch Behandlung mit einer Säure, vorzugsweise mit

3

# EP 0 174 525 B1

Dichloressigsäure in Dichlormethan, wird dann die Tritylgruppe abgespalten (Schritt 5. des Schemas). Das nun vollständig entschützte Oligonucleotid wird nach einem weiteren Waschschritt vom Adsorptionsmaterial eluiert. Das so vorgereinigte Oligonucleotid kann jetzt einfacher durch andere Reinigungsmethoden (z.B. Ionenaustauscher, Elektrophorese) in homogener Form isoliert werden (Schritt 7. des Schemas).

Beim erfindungsgemäßen Verfahren zur Aufreinigung von synthetischen Oligonucleotiden werden verschieden Lösungen durch ein geeignetes Adsorptionsmaterial durchgeleitet. Deshalb ist neben dem Handbetrieb eine Automatisierung möglich. Ein solcher Aufreinigungsapparat könnte im online-Betrieb mit DNA-Synthesemaschinen verwendet werden.

Das erfindungsgemäße Aufreinigungsverfahren wird am Beispiel eines Probegemisches (I) von 5'-Hydroxyoktanucleotid (II) und gereinigtem 5'-trityliertem Oktanucleotid (III) bewiesen.

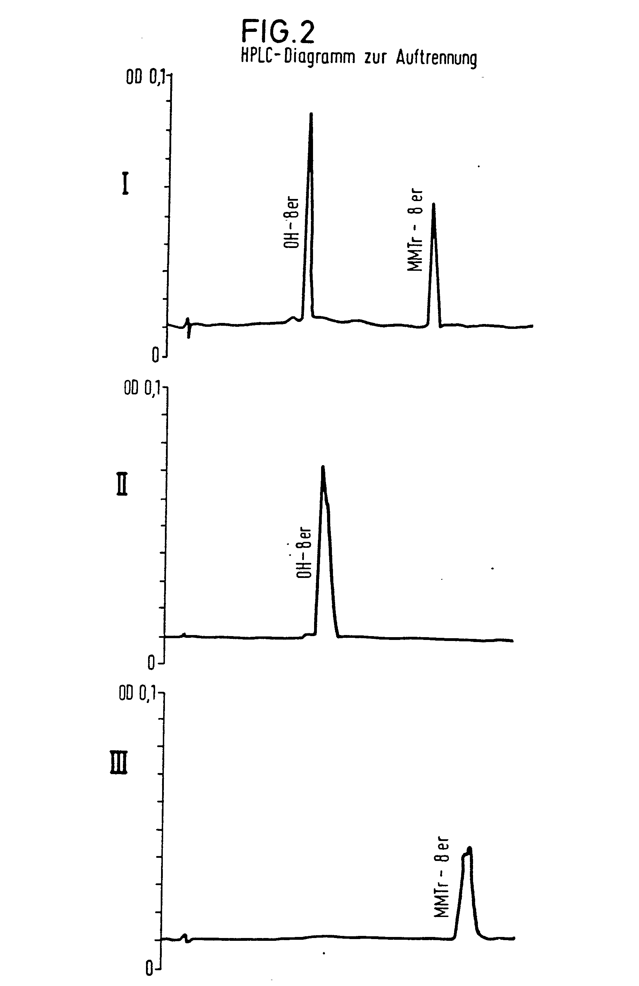

Fig. 2 zeigt die jeweiligen HPLC-Diagramme.

Die quantitative Detritylierung wird am Beispiel eines 5'-tritylierten Oktanucleotids bewiesen.

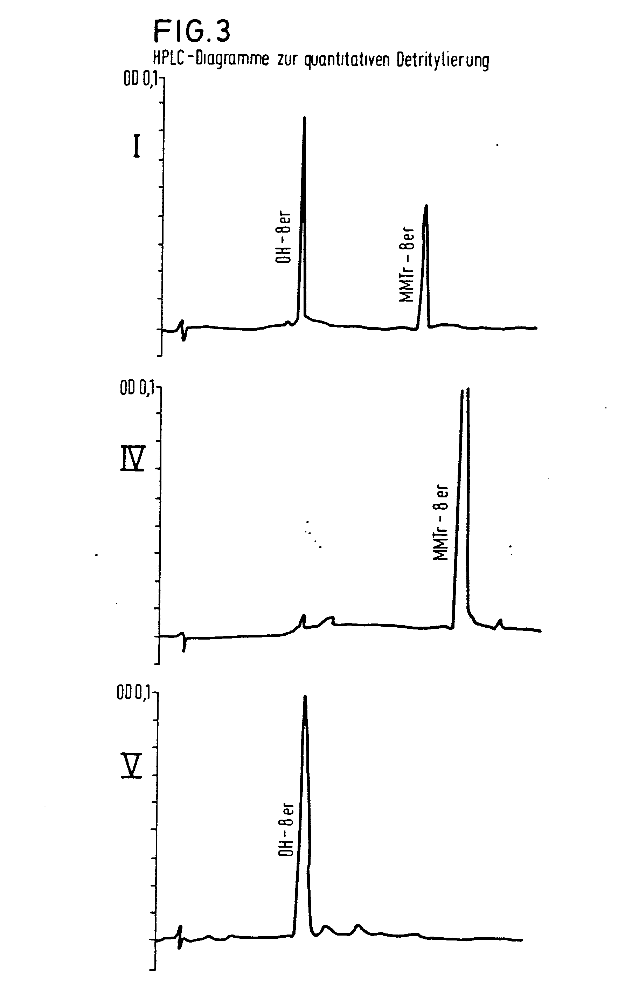

Fig. 3 zeigt die HPLC-Diagramme des Gemisches (I), des 5'-tritylierten Oktanucleotids (IV) und des Hydroxyoktanucleotids (V).

Die folgenden Versuche erläutern die Erfindung.

Hilfsmittel und Chemikalien:
— Einmal-Filtersäulen mit zweifacher 20 µm Fritte 6 ml Art. Nr. 71216 (Baker)
— Adapter für 6 ml Filtersäulen Art. Nr. 71220 (Baker)
— Druckluftanschluß
— Filtersäulenfüllmaterial: Polytetrafluorethylen=PTFE (ICI)
— DEAE-Cellulose DE 52 vorgequollen, Kat: No. 4057-050 (Whatman)
— Lösungsmittel:— 2M Triethylammoniumbicarbonat (pH 7,5)=2M TEAB (2mol/l TEAB)
— Ethanol absolut
— Dichlormethan=$CH_2 Cl_2$
— 3% Dichloressigsäure in Dichlormethan=DCA

Filtersäulen füllen:
1200 mg PTFE
200 mg DEAE-Cellulose
— im trockenen Zustand mischen
— zwischen die beiden Fritten in die Filtersäule füllen
— durch Druck auf die obere Fritte wird das Material zusammengepreßt
— Alle Lösungen werden mit Preßluft durch das Säulenmaterial gedrückt

Säule waschen und equilibrieren:
2 ml Ethanol
2 ml 100 mM (100 mmol/l) TEAB
2 ml 2M TEAB/Ethanol (4:1)
2 ml 50 mM TEAB

Beladen der Säule:
Das beladene Trägermaterial von einem 0,5 µmol Oligonucleotid-Syntheseansatz (Phosphotriestermethode) wird in 4 ml $NH_3$ (33%)/Pyridin (9:1) aufgenommen, 2 Tage bei Raumtemperatur und 12 Stunden bei 50°C im geschlossenen Gefäß stehengelassen. Nach Öffnen des Gefäßes wird das Ammoniak bei 50°C im Sandbad verdampft ($\approx$2h). Die restliche Lösung wird abpipettiert und das Trägermaterial mit 2 ml 50 mM TEAB und mit 2 ml 50 mM TEAB/Ethanol (1:1) gewaschen. Die drei Lösungen werden zusammengegeben und auf die Säule aufgetragen.

1. Waschen:
3×2 ml 50 mM TEAB
2 ml 100 mM TEAB

Auftrennung:
(Abtrennung der nicht tritylierten Komponenten) 2×2 ml 2M TEAB/Ethanol (95:5)

2. Waschen:
2 ml 50 mM TEAB
2 ml Ethanol
2 ml $CH_2Cl_2$

Säurebehandlung:
3% Dichloressigsäure in Dichlormethan wird 3 min lang durchgedrückt, dann mit
2 ml $CH_2Cl_2$

4

2 ml Ethanol
2 ml 50 mM TEAB gespült.

Eluieren:
2×2 ml 2M TEAB/Ethanol (4:1)

Einengen:
Die 4 ml Eluat werden im Vakuum bis zur Trockne eingeengt.
Der Rückstand wird dann weitergereinigt, entsprechend dem Schritt No. 7 des vorher beschriebenen Schemas.

Erklärung der HPLC-Diagramme:
Die erfindungsgemäße Auftrennung wird am Beispiel eines Gemisches aus gereinigtem 5'-tritylierten Oktanucleotid und 5'-Hydroxyoktanucleotid bewiesen.
HPLC-Diagramme I, II und III—
Trennung der Substanzen über eine $C_{18}$-Kieselgelsäule (Nucleosil® RPC18-Säule) 0,4×25 cm (Macherey-Nagel)
—Flußrate: 2 ml/min
—Gradient von 5% bis 50% Acetonitril in 0,1 M Triethylammoniumacetat (pH 6,3).
Quantitative Detritylierung am Beispiel eines 5'-tritylierten Oktanucleotids
—HPLC-Diagramme I, IV und V—
Trennung der Substanzen über eine RPC18-Säule (siehe oben).

Ausführungsbeispiel
Nach der Phosphortriestermethode am Cellulosefilter wird das Oligonucleotid GTT GTT TAA TGG GTA AAC synthetisiert. Als Bausteine wurden Dimethoxy-tritylierte, basengeschützte Phosphordiester eingesetzt (vgl. R. Frank, W. Heikens, G. Heiserberg-Moutsisis und H. Blöcker, in "Nucleic Acids Research", Bd. 11, S.4365 (1983)).
Das mit 0,5 µmol 18er-mer beladene Cellulosefilter wird in 4 ml $NH_3$ (33%)/Pyridin (9:1) aufgenommen, 2 Tage bei Raumtemperatur und 12 h bei 50°C im geschlossenen Gefäß stehengelassen. Nach Öffnen des Gefäßes wird das Ammoniak bei 50°C im Sandbad verdampft (etwa 2h). Die restliche Lösung wird abpipettiert und der Cellulosefilter mit 2 ml 50 mM TEAB und mit 2 ml 50 mM TEAB/Ethanol (1:1) gewaschen. Die drei Lösungen werden gesammelt und auf die Säule aufgegeben, die dann dreimal mit 2 ml 50 mM TEAB und einmal mit 2 ml 100 mM TEAB gewaschen wird. Durch zweimaliges Spülen der Säule mit je 2 ml 2 M TEAB/Ethanol (95:5) wird die nicht tritylierte Komponente abgetrennt. Die Säule wird mit 2 ml 50 mM TEAB, 2 ml Ethanol und 2 ml Dichlormethan gewaschen. Durch die Säule wird dann 3-proz. Dichloressigsäure in Dichlormethan 3 min lang durch gedrückt, worauf mit 2 ml Dichlormethan, 2 ml Ethanol und 2 ml 50 mM TEAB gespült wird. Die Säule wird zweimal mit je 2 ml 2M TEAB/Ethanol (4:1) eluiert.
Die 4 ml Eluat werden im Vakuum bis zur Trockene eingeengt. Der Rückstand wird durch Ionenaustauscher-HPLC-Chromatographie und Entsalzung (Schritt 7. des oben angegebenen Schemas) weitergereinigt.
Das Endprodukt des gereinigten 18er-mer hat eine optische Dichte von 1,1 bei 160 nm.

**Patentansprüche**

1. Verfahren zur Aufreinigung synthetischer Oligonucleotide, dadurch gekennzeichnet, daß man
—die gegebenenfalls von einem Träger abgespaltenen, noch mit lipophiler Schutzgruppe versehenen Oligonucleotide ohne Einengen der Abspaltungslösung
—an einen Träger mit Umkehrphaseneigenschaften und gebenenfalls Ionenaustauschereigenschaften adsorbiert, die Oligonucleotide ohne lipophile Schutzgruppe entfernt,
—die Schutzgruppe von den adsorbierten Oligonucleotiden abspaltet,
—die adsorbierten Oligonucleotide erneut wäscht,
—die adsorbierten Oligonucleotide vom Träger eluiert und
—danach die eluierten Oligonucleotide in an sich bekannter Weise weiter aufreinigt.
2. Verfahren nach Anspruch 1, dadurch gekennzeichnet, daß man als Träger mit Umkehrphaseneigenschaften Polytetrafluorethylen verwendet.
3. Verfahren nach Anspruch 1 oder 2, dadurch gekennzeichnet, daß man als Träger mit Ionenaustauschereigenschaften DEAE-Cellulose verwendet.
4. Verfahren nach einem der Ansprüche 1 bis 3, dadurch gekennzeichnet, daß man die wässrige Abspaltungslösung nach Abdampfen des Ammoniaks an den Träger adsorbiert.
5. Verfahren nach einem der Ansprüche 1 bis 4, dadurch gekennzeichnet, daß man die Abspaltungslösung auf eine mit dem Träger gefüllte Säule aufgibt und nacheinander mit einer Pufferlösung wäscht, mit einer Säure behandelt, mit einer Pufferlösung und einem organischen, mit Wasser mischbaren Lösungsmittel wäscht und vom Träger eluiert.

6. Verfahren nach einem der Ansprüche 1 bis 5, dadurch gekennzeichnet, daß man als Pufferlösung eine Triethylammoniumbicarbonatlösung verwendet.

7. Verfahren nach einem der Ansprüche 1 bis 6, dadurch gekennzeichnet, daß man als Säure Dichloressigsäure in Dichlormethan verwendet.

8. Verfahren nach einem der Ansprüche 1 bis 7, dadurch gekennzeichnet, daß man als organisches, mit Wasser mischbares Lösungsmittel Ethanol und/oder Dichlormethan verwendet.

9. Verfahren nach einem der Ansprüche 1 bis 8, dadurch gekennzeichnet, daß man mit Triethylammoniumbicarbonat und Ethanol im Verhältnis 4:1 eluiert.

## Revendications

1. Procédé de purification d'oligonucléotides synthétiques, caractérisé en ce que:
—on adsorbe les oligonucléotides séparés le cas échéant d'un support, encore munis d'un groupe protecteur lipophile, sans concentration de la solution d'élimination, sur un support ayant des propriétés de phase inversée et le cas échéant des propriétés d'échange d'ions,
—on élimine les oligonucléotides sans groupe protecteur lipophile,
—on élimine le groupe protecteur des oligonucléotides adsorbés,
—on lave à nouveau les oligonucléotides adsorbés,
—on élue les oligonucléotides adsorbés du support, puis
—on soumet les oligonucléotides élués à une nouvelle purification d'une manière connue en soi.

2. Procédé selon la revendication 1, caractérisé en ce qu'on utilise un polytétrafluoréthylène comme support ayant des propriétés de phase inversée.

3. Procédé selon la revendication 1 ou 2, caractérisé en ce qu'on utilise de la DEAE-cellulose comme support ayant des propriétés d'échange d'ions.

4. Procédé selon l'une des revendications 1 à 3, caractérisé en ce qu'on adsorbe sur le support la solution aqueuse d'élimination après évaporation de l'ammoniac.

5. Procédé selon l'une des revendications 1 à 4, caractérisé en ce qu'on verse la solution d'élimination sur une colonne remplie du support, en ce que, successivement, on la lave avec une solution tampon, en ce qu'on la traite par un acide, on la lave avec une solution tampon et un solvant organique miscible à l'eau, et on l'élue du support.

6. Procédé selon l'une des revendications 1 à 5, caractérisé en ce qu'on utilise comme solution tampon une solution de bicarbonate de triéthylammonium.

7. Procédé selon l'une des revendications 1 à 6, caractérisé en ce qu'on utilise comme acide de l'acide dichloracétique dans du dichlorométhane.

8. Procédé selon l'une des revendications 1 à 7, caractérisé en ce qu'on utilise, comme solvant organique miscible à l'eau, de l'éthanol et/ou du dichlorométhane.

9. Procédé selon l'une des revendications 1 à 8, caractérisé en ce qu'on élue avec du bicarbonate de triéthylammonium et de l'éthanol dans le rapport 4:1.

## Claims

1. Process for the purification of synthetic oligonucleotides, characterised in that
—the ologonucleotides, optionally cleaved from a carrier and still provided with a lipophilic protecting group, are, without concentration of the cleavage solution,
—adsorbed onto a carrier having reverse-phase properties and optionally ion-exchanger properties, and the oligonucleotides without a lipophilic protecting group are removed,
—the protecting group is removed from the adsorbed oligonucleotides,
—the adsorbed oligonucleotides are washed again,
—the adsorbed oligonucleotides are eluted from the carrier and
—the eluted oligonucleotides are then purified further in a manner known *per se*.

2. Process according to claim 1, characterised in that polytetrafluoroethylene is used as the carrier having reverse-phase properties.

3. Process according to claim 1 or 2, characterised in that DEAE-cellulose is used as the carrier having ion-exchanger properties.

4. Process according to any one of claims 1 to 3, characterised in that after evaporating off the ammonia the aqueous cleavage solution is adsorbed onto the carrier.

5. Process according to any one of claims 1 to 4, characterised in that the cleavage solution is introduced onto a column filled with the carrier and, in succession, washed with a buffer solution, treated with an acid, washed with a buffer solution and an organic water-miscible solvent and eluted from the carrier.

6. Process according to any one of claims 1 to 5, characterised in that a triethylammonium bicarbonate solution is used as the buffer solution.

7. Process according to any one of claims 1 to 6, characterised in that dichloroacetic acid in dichloromethane is used as the acid.

8. Process according to any one of claims 1 to 7, characterised in that ethanol and/or dichloromethane is used as the organic water-miscible solvent.

9. Process according to any one of claims 1 to 8, characterised in that the elution is carried out with triethylammonium bicarbonate and ethanol in a ratio of 4:1.

**FIG.1 :** Schema der chemischen DNA - Synthese
an einem polymeren Träger

teilgeschutztes
Ausgangsnucleotid

polymerer Träger

Stufe 1 : Verknüpfung

Stufe 2 : Selektive
Deblockierung

teilgeschutzter
Nucleotid-Aufbaublock

Stufe 3 : Kettenverlängerung

cyclische Wiederholung
der Stufen 2 und 3

□Tritylschutzgruppe

# FIG.2
HPLC-Diagramm zur Auftrennung

# FIG.3
HPLC-Diagramme zur quantitativen Detritylierung